# EUROPEAN PATENT APPLICATION

(11) **EP 3 970 770 A1**
(43) Date of publication of application: **23.03.2022**
(21) Application number: 20805624.2
(22) Date of filing: 14.05.2020
(51) Int. Cl.: A61M 5/42, A61B 5/153

(54) **PUNCTURE-ASSISTANCE TOOL, PUCTURE DEVICE, AND PUNCTURE METHOD**

(30) Priority: 14.05.2019 JP 2019091579
(71) Applicant: The School Corporation Kansai University, Suita-shi, Osaka 564-8680 (JP); Aiki Riotech Corporation, Inazawa-shi, Aichi 492-8162 (JP)
(72) Inventor: AOYAGI, Seiji, Suita-shi, Osaka 564-8680 (JP); MATSUMOTO, Hajime, Inazawa-shi, Aichi 492-8162 (JP)
(74) Representative: Vidon Brevets & Stratégie
(86) International application number: PCT/JP2020/019269
(87) International publication number: WO 2020/230848

(57) **Abstract**

The present invention has an object to cause no pain to a living body and prevent a needle from being buckled. A puncture assisting tool (10) includes: a frame 11 that is attached to skin (S); and a guide part (12) that is located inside a region of the frame (11) in which region the frame (11) is attached to the skin, the guide part (12) being provided with a through-hole (12a) through which a needle passes and guiding movement of the needle.

## Description

### Technical Field

The present invention relates to, for example, a puncture assisting tool that is used to insert a needle into the skin of a living body.

### Background Art

In the collection of blood or the like of a living body, a puncture assisting tool is used to insert a needle into the skin of the living body. For example, Patent Literature 1 discloses a puncture instrument that includes a suction cup, a gripping part, a cavity, a nonreturn valve, and an injection needle. In the technique disclosed in Patent Literature 1, by lifting the gripping part up, a negative pressure is generated inside the cavity so that the suction cup suction-attracts the skin. Then, by releasing the gripping part, the injection needle is inserted into the skin.

### Citation List

### [Patent Literature]

[Patent Literature 1]
Japanese Patent Application Publication Tokukai No. 2009-112416

### Summary of Invention

### Technical Problem

Unfortunately, the technique of Patent Literature 1 causes the skin to be greatly depressed through the application of pressure by the injection needle during the insertion of the injection needle. This stimulates a pain sensory nerve of a living body and thus causes pain to the living body. Further, the technique of Patent Literature 1 may cause the needle to be buckled during the insertion of the needle.

An aspect of the present invention has an object to achieve a puncture assisting tool that makes it possible to cause no pain to a living body and prevent a needle from being buckled.

### Solution to Problem

In order to attain the object, a puncture assisting tool in accordance with an aspect of the present invention includes: an attachment part that is attached to skin; and a guide part that is located inside a region of the attachment part in which region the attachment part is attached to the skin, the guide part being provided with a through-hole through which a needle passes and guiding movement of the needle.

### Advantageous Effects of Invention

An aspect of the present invention makes it possible to cause no pain to a living body and prevent a needle from being buckled.

### Brief Description of Drawings

Fig. 1 is a view illustrating a configuration of a puncture instrument in accordance with Embodiment 1 of the present invention.
Fig. 2 is a perspective view of a puncture assisting tool which is included in the puncture instrument.
Fig. 3 is a perspective view of a puncture assisting tool cut along a plane that passes through a guide part and an air discharge part which are included in the puncture assisting tool.
Fig. 4 illustrates the puncture assisting tool as viewed from an upward direction in Fig. 2.
Fig. 5 is a cross-sectional view taken along the line A-A illustrated in Fig. 4.
   (a) to (c) of Fig. 6 are views each for describing an example of use of the puncture instrument.
Fig. 7 is a view illustrating a state in which skin of a living body has been sucked by the puncture assisting tool.
Fig. 8 is an enlarged view illustrating a state in which a needle is inserted into the living body.
   (a) of Fig. 9 is a plan view of a puncture assisting tool in accordance with Embodiment 2 of the present invention, and (b) of Fig. 9 is a side view of the puncture assisting tool.
Fig. 10 is a view illustrating a configuration of a puncture instrument in accordance with Embodiment 3 of the present invention.
   (a) to (c) of Fig. 11 are views each for describing an example of use of the puncture instrument.
Fig. 12 is a view illustrating a configuration of a puncture instrument in accordance with Embodiment 4 of the present invention.
Fig. 13 is a perspective view of a puncture assisting tool in accordance with Embodiment 5 of the present invention.
Fig. 14 illustrates the puncture assisting tool as viewed from an upward direction in Fig. 13.
Fig. 15 is a view for describing the structure of a first fixing part included in the puncture assisting tool and is a cross-sectional view taken along the line B-B illustrated in Fig. 14.
Fig. 16 is a cross-sectional view taken along the line C-C of Fig. 14 and illustrating a state in which pressure inside a frame of an attachment part is reduced during puncture in which the puncture assisting tool is used.

### Description of Embodiments

### [Embodiment 1]

The following description will discuss an embodiment of the present invention in detail.

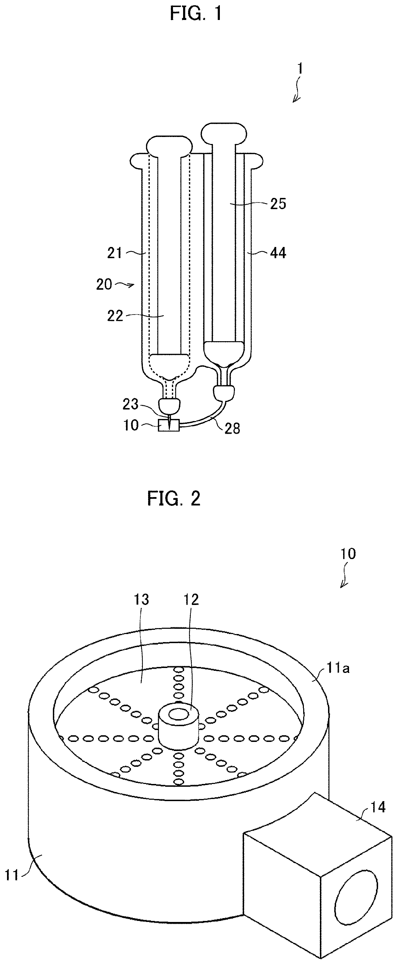

Fig. 1 is a view illustrating a configuration of a puncture instrument 1 in accordance with Embodiment 1. As illustrated in Fig. 1, the puncture instrument 1 includes a puncture assisting tool 10, a double syringe barrel 20, and a tube 28 (air flow part).

### (Puncture assisting tool 10)

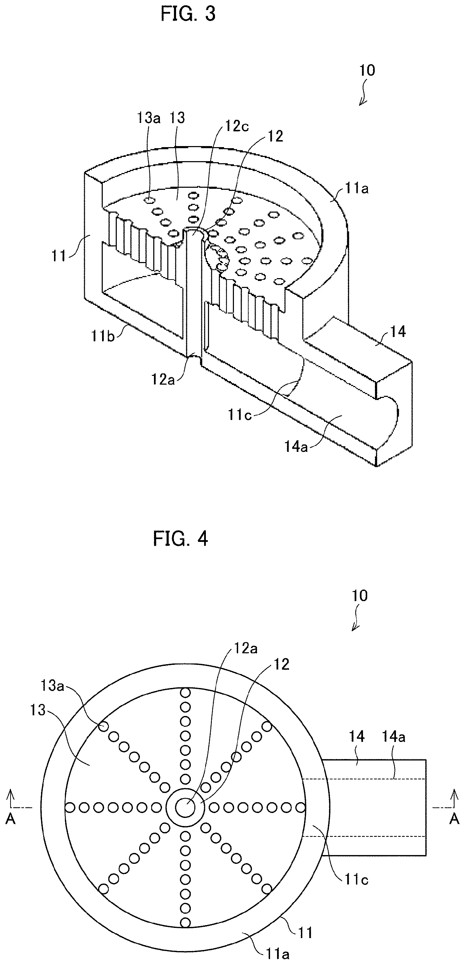

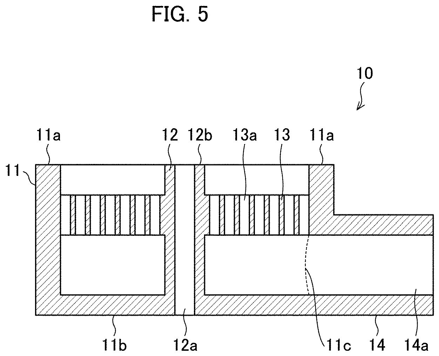

Fig. 2 is a perspective view of the puncture assisting tool 10. Fig. 3 is a perspective view of the puncture assisting tool 10 cut along a plane that passes through a guide part 12 and an air discharge part 14 (described later). Fig. 4 illustrates the puncture assisting tool 10 as viewed from an upward direction in Fig. 2. Fig. 5 is a cross-sectional view taken along the line A-A illustrated in Fig. 4.

As illustrated in Figs. 2 to 5, the puncture assisting tool 10 includes a frame 11, the guide part 12, a support 13, and the air discharge part 14.

The frame 11 has a cylindrical shape. The frame 11 includes: a first end 11a, which is one end of a cylinder and is an end on a side that contacts the skin of a living body; and a second end 11b, which is another end of the cylinder. The first end 11a has a circular shape and has an opening on the inside, and the second end 11b is made of a flat plate. Thus, the frame 11 has a shape in which a face thereof on the side that contacts the skin of a living body is open.

Further, the frame 11 includes an opening 11c formed on a side surface of the cylinder and in a region where the air discharge part 14 (described later) is installed.

The guide part 12 is a member for guiding movement of a needle 23 (described later). The guide part 12 has a cylindrical shape. The cylindrical shape is coaxial with the cylindrical shape of the frame 11. The guide part 12 has a through-hole 12a which is formed along an axial direction (vertical direction in Fig. 5) of the cylindrical shape and through which the needle 23 passes. The guide part 12 is formed such that an inner diameter of the guide part 12 (in other words, an inner diameter of the through-hole 12a) is slightly larger than an outer diameter of the needle 23 (described later). For example, the guide part 12 may be formed such that the inner diameter of the guide part 12 is 1.05 to 1.2 times larger than the outer diameter of the needle 23.

Further, as illustrated in Fig. 5, a third end 12b, which is an end of the guide part 12 on the side that contacts the skin of a living body, is located on the same plane as the first end 11a of the frame 11 in the vertical direction in Fig. 5.

The support 13, in an inner part of the frame 11, is formed between the first end 11a and the second end 11b in the axial direction (vertical direction in Fig. 2) of the cylindrical shape of the frame 11. The support 13 is made of a circular flat plate having a diameter of the same length as an inner diameter of the frame 11. As illustrated in Figs. 2 to 5, the support 13 has a plurality of air intake holes 13a formed therein that penetrate in the axial direction of the cylindrical shape of the frame 11. Note that although Figs. 2 to 5 differ in number of the air intake holes 13a formed in the support 13, the number (density) of the air intake holes 13a formed in the support 13 is not particularly limited, and can be set as appropriate, provided that the strength of the support 13 is not greatly decreased.

The air discharge part 14 connects a space in the inner part of the frame 11 and the tube 28 to each other. The air discharge part 14 is provided on the side surface of the frame 11. The air discharge part 14 has a cylindrical hole 14a formed therein. The hole 14a is connected at one end thereof to the opening 11c, which is provided in the frame 11. The hole 14a is connected at another end thereof to the tube 28, as illustrated in Fig. 1.

### (Double syringe barrel 20)

As illustrated in Fig. 1, the double syringe barrel 20 includes a first syringe 21, a first plunger 22, the needle 23, a second syringe 24 (suction device), and a second plunger 25. The double syringe barrel 20 is a syringe barrel that includes two syringes which are the first syringe 21 and the second syringe 24.

The first syringe 21, the first plunger 22, and the needle 23 are the ones for collecting blood from a living body. The first syringe 21 is stored inside the first plunger 22.

The needle 23 is attached to a tip of the first plunger 22. It is better that the outer diameter of the needle 23 is as small as possible to reduce the likelihood of causing pain to a living body. The needle 23 is hollow inside.

With the above configuration, by pulling up (pulling out) the first plunger 22, it is possible to collect blood of a living body through the needle 23 which is inserted into the skin of the living body.

The second syringe 24 and the second plunger 25 serve as a suction device for sucking air in the inner part of the frame 11 of the puncture assisting tool 10. The second plunger 25 is stored inside the second syringe 24. The second syringe 24 is connected at a tip thereof to another end of the tube 28, which another end is on the other side of the tube 28 from one end which is connected to the air discharge part 14 of the puncture assisting tool 10.

With the above configuration, by pulling up the second plunger 25 inside the second syringe 24, air in the inner part of the frame 11 of the puncture assisting tool 10 is sucked, so that pressure in the inner part of the frame 11 can be reduced.

### (Example of use of puncture instrument 1)

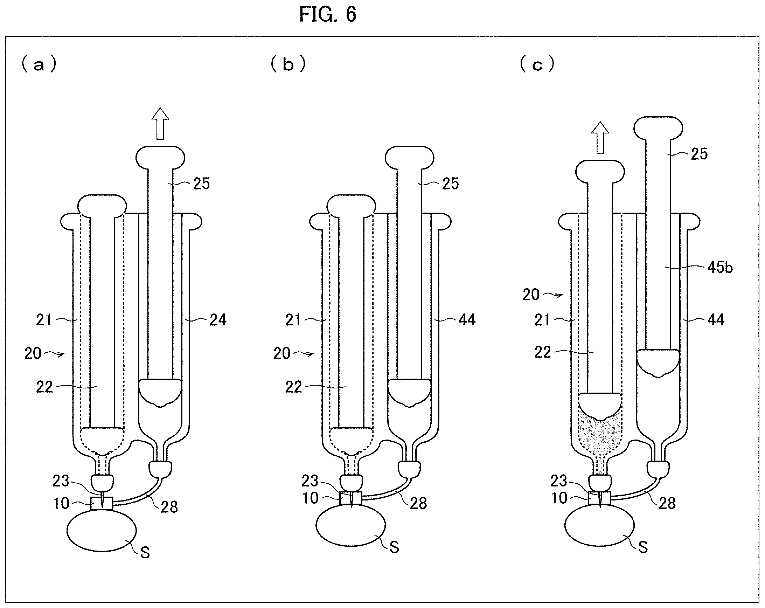

Next, an example of use of the puncture instrument 1 will be described. (a) to (c) of Fig. 6 are views for describing an example of use of the puncture instrument 1 (example of a puncture method).

In the use of the puncture instrument 1, first of all, the first plunger 22 and the second plunger 25 are located at the lowermost parts of the first syringe 21 and the second syringe 24, respectively, as illustrated in Fig. 1.

Next, the puncture assisting tool 10 is brought into contact with the skin S of a living body. In this state, as illustrated in (a) of Fig. 6, the second plunger 25 is pulled up. This sucks air in the inner part of the frame 11 of the puncture assisting tool 10 through the tube 28 and reduces pressure in the inner part of the frame 11. Note that since the support 13 has the plurality of air intake holes 13a formed therein, it is possible to reduce the pressure in the whole inner part of the frame 11.

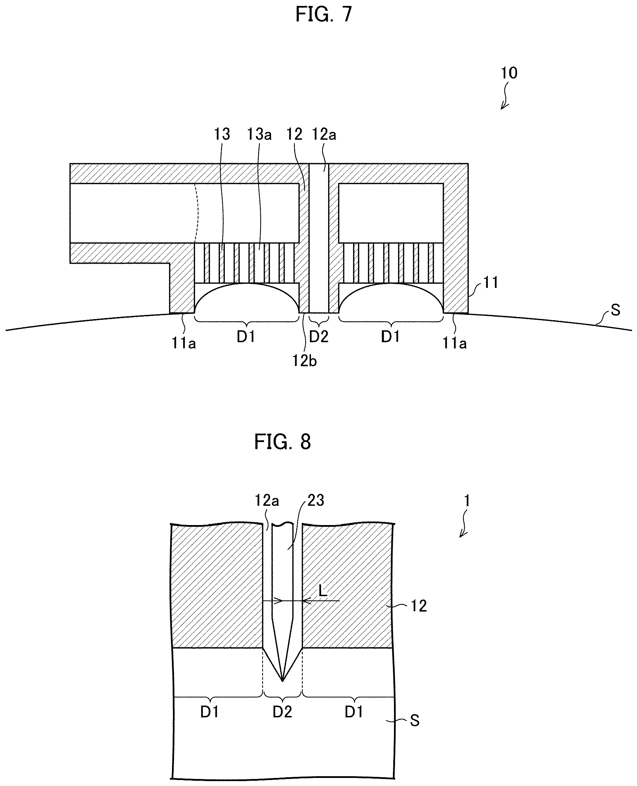

Fig. 7 is a view illustrating a state in which the skin S of the living body has been sucked by the puncture assisting tool 10. When the pressure in the inner part of the frame 11 is reduced, a region D1 of the skin S of the living body with which region D1 the puncture assisting tool 10 makes contact (more specifically, a region corresponding to the inner part of the frame 11) is sucked toward the inner part of the frame 11, as illustrated in Fig. 7. This brings the skin S in the region D1 into a state of being attached to (in other words, a state of being suction-attracted to) the puncture assisting tool 10 (more specifically, the inner part of the frame 11). In other words, the inner part of the frame 11 serves as an attachment part that is attached to the skin S.

Next, as illustrated in (b) of Fig. 6, by moving the double syringe barrel 20 toward the skin of the living body, the needle 23 is inserted into the skin S of the living body. At this time, while the inner part of the frame 11 continues to be in the state of being attached to the skin S, the needle 23 is inserted into the skin S. Further, when the needle 23 is inserted into the skin S, the movement of the needle 23 is guided by the guide part 12 of the puncture assisting tool 10. Further, as described earlier, the cylindrical shape of the guide part 12 is coaxial with the cylindrical shape of the frame 11. In other words, the guide part 12 is provided inside the region D1 in which the skin S of the living body is sucked. Thus, a place where the needle 23 is inserted into the skin S of the living body is a region (region D2 illustrated in Fig. 7) of the skin S inside the region D1 which is attached to the puncture assisting tool 10.

Fig. 8 is an enlarged view illustrating a state in which the needle 23 is inserted into the skin S of the living body. As described above, the above region D1 in the skin S is in a state of being attached to the puncture assisting tool 10. Thus, as illustrated in Fig. 8, the above region D1 of the skin S is not depressed when the needle 23 is inserted into the skin S, and the depressed spot of the skin S due to the insertion is only the above region D2 of the skin S. This results in decrease in distance (distance L illustrated in Fig. 8) between the center of the needle 23 and an inner surface of the through-hole 12a of the guide part 12 in the direction (transverse direction illustrated in Fig. 8) perpendicular to a direction in which the needle 23 is inserted into the skin S of the living body. As a result, it is possible to increase shear stress applied to the skin S by the needle 23. This makes it possible to insert the needle 23, which is easily buckled, into the skin S without causing the buckling of the needle 23.

Further, as described earlier, to guide the needle 23, the guide part 12 is formed such that the inner diameter of the guide part 12 (in other words, the inner diameter of the through-hole 12a) is slightly larger than the outer diameter of the needle 23. Thus, the distance L is slightly smaller than the outer diameter of the needle 23. This makes it possible to increase the shear stress applied to the skin S by the needle 23.

Further, since the needle 23 is guided by the guide part 12 (in other words, supported by the guide part 12) during the insertion, it is possible to prevent buckling of the needle 23.

Then, after the needle 23 has been inserted into the skin S of the living body, the first plunger 22 is pulled up as illustrated in (c) of Fig. 6. In this way, by sucking blood of the living body through the needle 23, the blood of the living body is collected.

As described above, the puncture assisting tool 10 in Embodiment 1 includes: the frame 11 that suction-attracts the skin S of the living body under a reduced pressure inside the frame 11; and the guide part 12 that is located in the inner part of the frame 11 which inner part is a region of the frame 11 to which region the skin S is attached, the guide part 12 being provided with the through-hole 12a through which the needle 23 passes and guiding movement of the needle 23.

According to the above configuration, it is possible to insert the needle 23 into the skin S in the state in which the skin S of the living body is suction-attracted to the inner part of the frame 11. This makes it possible to increase the shear stress applied to the skin S by the needle 23. As a result, it is possible to insert the needle 23, which is easily buckled, into the skin S without causing the buckling of the needle 23. Further, since the needle 23 is guided by the guide part 12 during the insertion, it is possible to prevent buckling of the needle 23. In other words, it is possible to use the needle 23 having a small outer diameter. As a result, it is possible to prevent stimulation of pain spots of a living body.

Further, in the puncture assisting tool 10, the third end 12b, which is an end of the guide part 12 on the side that contacts the skin of a living body, is located on the same plane as the first end 11a of the frame 11 in the vertical direction in Fig. 5. In other words, the position of the first end 11a, which is a skin-side end of the frame 11, and the position of the third end 12b, which is a skin-side end of the guide part 12, are aligned with each other. Thus, it is possible to reliably suction-attract the skin S to between the first end 11a of the frame 11 and the third end 12b of the guide part 12. This, as a result, allows a depressed spot of the skin S by the puncture to be reliably only the above-described region D2 of the skin S. However, in the puncture assisting tool of an aspect of the present invention, the third end 12b of the guide part 12 may be located slightly inside the frame 11 with respect to the first end 11a of the frame 11.

Further, the puncture assisting tool 10 includes, as the attachment part that causes the skin S to be attached to the puncture assisting tool 10, the support 13 for supporting the skin S that has been sucked into the inner part of the frame 11. Further, as illustrated in Fig. 5, the support 13 is provided so as to be closer to a living body skin side (upper side in Fig. 5) than to the air discharge part 14 in the axial direction (vertical direction in Fig. 5) of the cylindrical shape of the frame 11. According to the above configuration, the skin S sucked into the inner part of the frame 11 is supported by the support 13, as illustrated in Fig. 7. As a result, it is possible to prevent the air discharge part 14 from being covered by the skin S. That is, it is possible to prevent air intake of the second syringe 24 from being hampered. Thus, it is possible to continuously suction-attract the skin S with respect to the puncture assisting tool 10. Note that a puncture assisting tool of an aspect of the present invention may be configured not to include the support 13.

Note that instead of the support 13, a plurality of columnar supports may be formed inside the frame 11 (on the second end 11b). The plurality of columnar supports support the skin S and can take in air from a space between the respective plurality of columnar supports.

Note that although the puncture instrument 1 of Embodiment 1 is configured to include the double syringe barrel 20 that has the first syringe 21 for collecting blood from a living body and the second syringe 24 as a suction device for sucking air in the inner part of the frame 11 of the puncture assisting tool 10, the present invention is not limited to this configuration. A puncture instrument of an aspect of the present invention may be configured to include a first syringe 21 and a second syringe 24 which are separated from each other. Further, the suction device for sucking air in the inner part of the frame 11 of the puncture assisting tool 10 is not limited to a suction device that uses a syringe, and can be, for example, a pump or any other suction device that is capable of sucking air. In addition, a puncture assisting tool of an aspect of the present invention can be the one that includes the puncture assisting tool 10 and the above-described suction device. However, in Embodiment 1, a mechanism such as a pump as a suction device is not required. This makes it possible to reduce production costs.

In addition, the puncture instrument 1 of Embodiment 1 uses the hollow needle 23 to collect blood of a living body. However, the present invention is not limited to such a configuration. For example, an aspect can be employed in which after a needle, for which a solid needle is used, is inserted into a living body and is then removed from the living body, blood coming from the skin of the living body is collected.

### [Embodiment 2]

The following description will discuss another embodiment of the present invention. Note that for convenience of description, members having functions identical to those described in Embodiment 1 are assigned identical referential numerals, and their descriptions are omitted here.

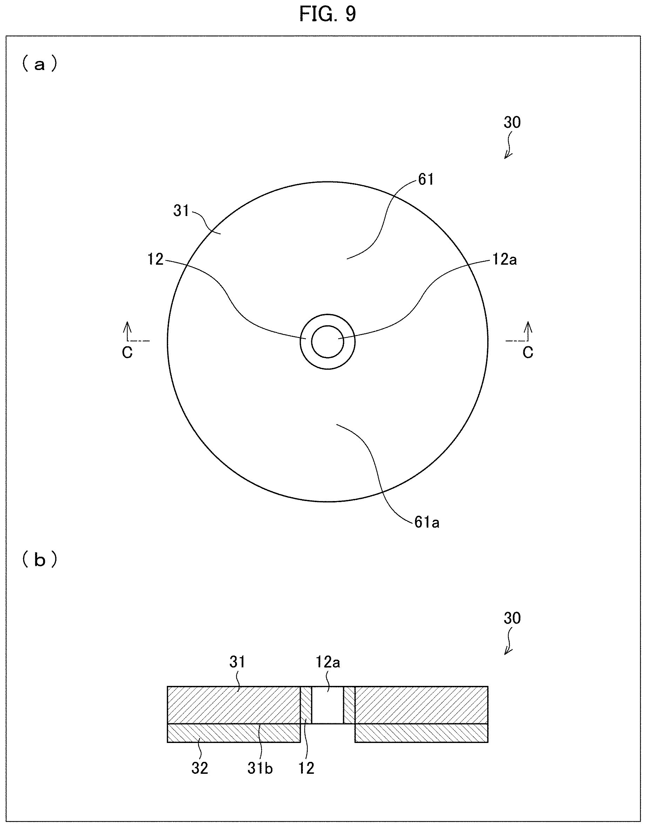

(a) of Fig. 9 is a plan view of a puncture assisting tool 30 of Embodiment 2, and (b) of Fig. 9 is a side view of the puncture assisting tool 30. As illustrated in (a) and (b) of Fig. 9, the puncture assisting tool 30 includes a cylindrical member 31 and a guide part 12.

The cylindrical member 31 has a cylindrical shape and has a through-hole 31a which is formed such that the guide part 12 is formed along a central axis thereof. An adhesive 32 (e.g., mucin or the like) (attachment part) is affixed to a lower surface 31b of the cylindrical member 31.

In a puncture method of Embodiment 2, first of all, the lower surface 31b of the puncture assisting tool 30 is brought into contact with skin S of a living body. This causes the adhesive 32 affixed to the lower surface 31b to stick to the skin S.

Next, a needle 23 placed at a tip of a syringe (not illustrated) is inserted into the skin S of the living body. At this time, while the adhesive 32 continues to be in the state of being attached to the skin S, the needle 23 is inserted into the skin S. Further, when the needle 23 is inserted into the skin S, the movement of the needle 23 is guided by the guide part 12 of the puncture assisting tool 30. Further, the cylindrical shape of the guide part 12 is coaxial with the cylindrical shape of the cylindrical member 31. In other words, the guide part 12 is provided inside the lower surface 31b to which the skin S of the living body sticks when viewed from a downward direction in (b) of Fig. 9. Thus, a place where the needle 23 is inserted into the skin S of the living body is a region of the skin S inside a region which is attached to the puncture assisting tool 30.

As described above, in Embodiment 2, as in Embodiment 1, it is possible to insert the needle 23 into the skin S in a state in which the region of the skin S other than a region corresponding to the inside of the guide part 12 is attached to the puncture assisting tool 30. Moreover, since a distance is small between the center of the needle 23 and an inner surface of the through-hole 12a of the guide part 12 in the direction perpendicular to a direction in which the needle 23 is inserted into the skin S of the living body, it is possible to increase shear stress applied to the skin S by the needle 23. This makes it possible to insert the needle 23, which is easily buckled, into the skin S without causing the buckling of the needle 23. Further, since the needle 23 is guided by the guide part 12 (in other words, supported by the guide part 12) during the insertion, it is possible to prevent buckling of the needle 23.

In Embodiment 2, the adhesive 32 is used to cause the skin S to stick to the puncture assisting tool 30. Note, however, that another method (e.g., an adhesive agent or the like) can be alternatively used to cause the skin S to be attached to the puncture assisting tool 30.

### [Embodiment 3]

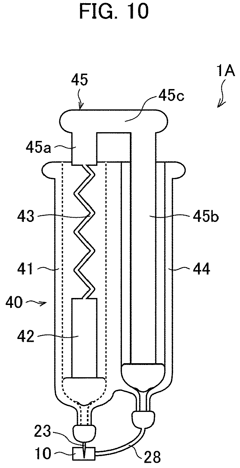

Fig. 10 is a view illustrating a configuration of a puncture instrument 1A of Embodiment 3. As illustrated in Fig. 10, the puncture instrument 1A includes a double syringe barrel 40 instead of the double syringe barrel 20 of Embodiment 1.

As illustrated in Fig. 10, the double syringe barrel 40 includes a first syringe 41, a first piston 42, a string 43 (connecting member), a needle 23, a second syringe 44, and a second piston 45. The double syringe barrel 40 is a syringe barrel that includes two syringes which are the first syringe 41 and the second syringe 44.

The first piston 42 is stored inside the first syringe 41. The first piston 42 is such that the length of the first piston 42 in a direction along which the first piston 42 moves (length in the vertical direction in Fig. 10) is a length such that, when one end of the first piston 42 on a needle 23 side is located at a position closest to the needle 23, the other end of the first piston 42 which is opposite the one end thereof on the needle 23 side is stored inside the first syringe 41.

The second piston 45 includes a third plunger 45a, a fourth plunger 45b, and a connecting part 45c for connecting one end of the third plunger 45a and one end of the fourth plunger 45b. The third plunger 45a is a plunger corresponding to the first syringe 41, and the fourth plunger 45b is a plunger corresponding to the second syringe 44. The third plunger 45a is shorter than the fourth plunger 45b in length in the vertical direction in Fig. 10.

The string 43 has one end that is connected to an upper end of the first piston 42 and the other end that is connected to a lower end of the third plunger 45a. The length of the string 43 is greater than a distance between the upper end of the first piston 42 and the lower end of the third plunger 45a when the second piston 45 is in the lowest position (i.e., when the fourth plunger 45b contacts the lower end of the second syringe 44).

### (Example of use of puncture instrument 1A)

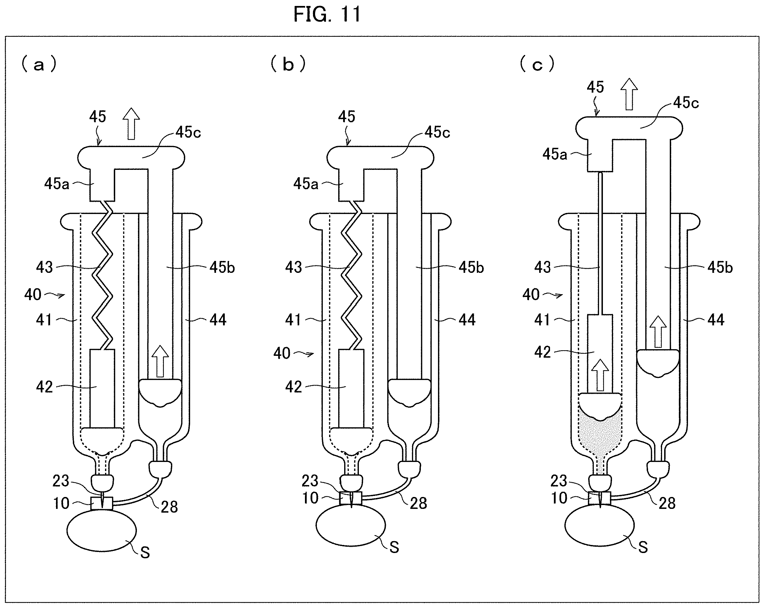

Next, an example of use of a puncture instrument 1A will be described. (a) to (c) of Fig. 11 are views for describing an example of use of the puncture instrument 1A (example of a puncture method).

In the use of the puncture instrument 1A, first of all, the first piston 42 and the second piston 45 are located at the lowermost parts of the first syringe 21 and the second syringe 24, respectively, as illustrated in Fig. 10. In this state, as described above, the length of the string 43 is greater than the distance between the upper end of the first piston 42 and the lower end of the third plunger 45a when the second piston 45 is in the lowermost position. Thus, the string 43 is in a loose state.

Next, the puncture assisting tool 10 is brought into contact with skin S of a living body. In this state, as illustrated in (a) of Fig. 11, the second piston 45 is pulled up. Thus, the third plunger 45a and the fourth plunger 45b are pulled upward. By pulling up the fourth plunger 45b, air in the inner part of the frame 11 of the puncture assisting tool 10 is sucked through the tube 28, and pressure in the inner part of the frame 11 is reduced. This allows the inner part of the frame 11 to be attached to the skin S of the living body.

On the other hand, since the string 43 is in a loose state even when the third plunger 45a is pulled up, the first piston 42 is not pulled up.

Next, as illustrated in (b) of Fig. 11, by moving the double syringe barrel 40 toward the skin of the living body, the needle 23 is inserted into the skin S of the living body. At this time, while the inner part of the frame 11 continues to be in the state of being attached to the skin S, the needle 23 is inserted into the skin S. Further, when the needle 23 is inserted into the skin S, the movement of the needle 23 is guided by the guide part 23 of the puncture assisting tool 10.

Next, as illustrated in (c) of Fig. 11, the second piston 45 is further pulled up. This brings the string 43 into a stretched state and applies tension to the string 43. From this condition, by further pulling up the second piston 45, the first piston 42 is pulled up through the string 43. In this way, by sucking blood of the living body through the needle 23, it is possible to collect the blood of the living body.

### [Embodiment 4]

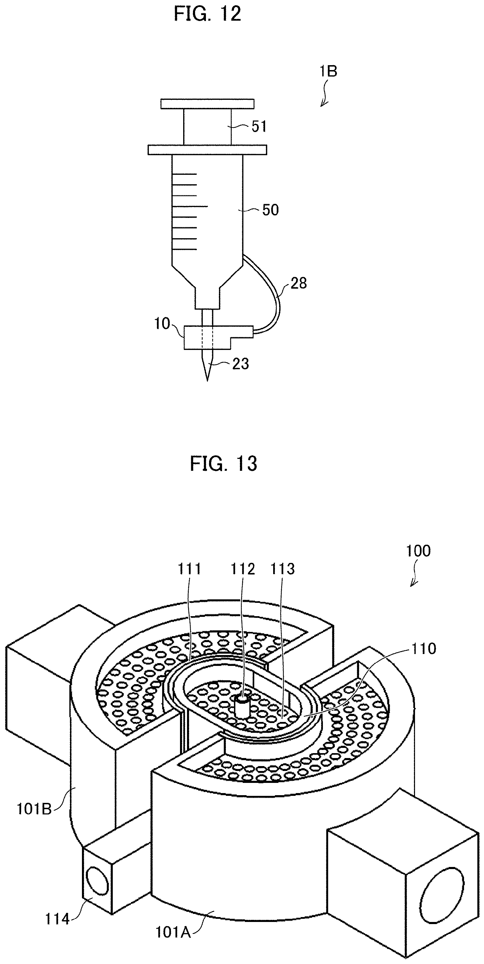

Fig. 12 is a view illustrating a configuration of a puncture instrument 1B of Embodiment 4. As illustrated in Fig. 12, the puncture instrument 1B includes a syringe 50 and a plunger 51 instead of the double syringe barrel 20 of Embodiment 1. In Embodiment 4, a tube 28 is connected to a space in an inner part of the syringe 50.

In a puncture method of Embodiment 4, first of all, a puncture assisting tool 10 is brought into contact with skin S of a living body. In this state, the plunger 51 is pulled up. This sucks air in the inner part of the frame 11 of the puncture assisting tool 10 through the tube 28 and reduces pressure in the inner part of the frame 11.

Next, the syringe 50 is moved toward the skin S of the living body so that a needle 23 is inserted into the skin S of the living body. At this time, while the inner part of the frame 11 continues to be in the state of being attached to the skin S, the needle 23 is inserted into the skin S. Further, when the needle 23 is inserted into the skin S, the movement of the needle 23 is guided by the guide part 12 of the puncture assisting tool 10.

Next, the plunger 51 is further pulled up. In this way, by sucking blood of the living body through the needle 23, the blood of the living body is collected.

As described above, the puncture instrument 1B of Embodiment 4 includes the puncture assisting tool 10, the syringe 50 having the needle 23, and the tube 28 that connects the space in the inner part of the frame 11 of the puncture assisting tool 10 and the inner part of the syringe 50 to each other. The above configuration enables, with use of a single syringe 50, sucking of air in the space in the inner part of the frame 11 and collection of blood of a living body. This makes it possible to simplify the configuration of the puncture instrument 1B and reduce the production costs.

### [Embodiment 5]

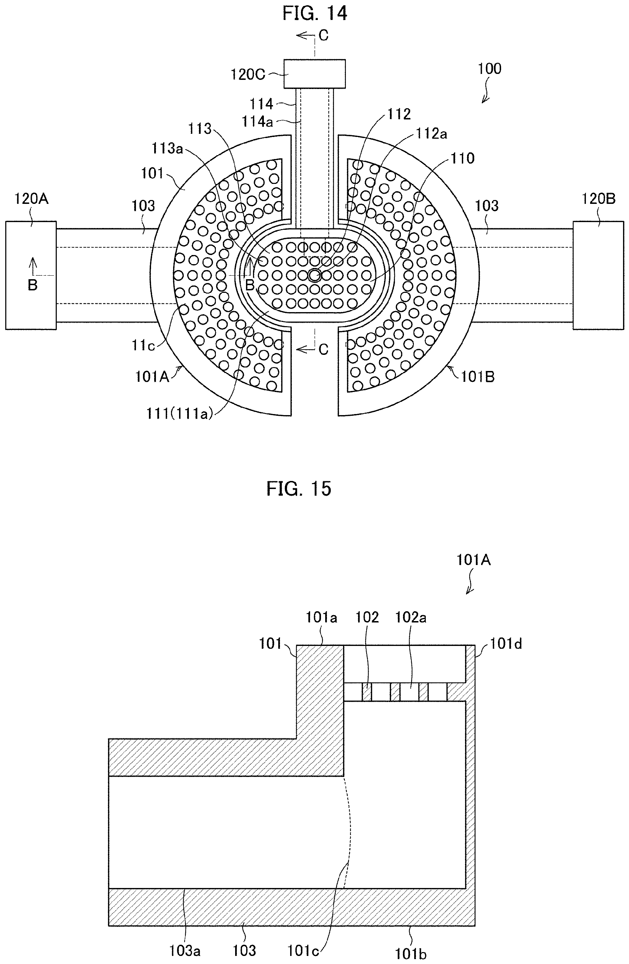

Fig. 13 is a perspective view of a puncture assisting tool 100 of Embodiment 5. Fig. 14 illustrates the puncture assisting tool 100 as viewed from an upward direction in Fig. 13.

As illustrated in Figs. 13 and 14, the puncture assisting tool 100 includes a first fixing part 101A, a second fixing part 101B, an attachment part 110, a pump 120A, a pump 120B, and a pump 120C (suction device). Note that in Fig. 13, the pumps 120A to 120C are not illustrated.

The first fixing part 101A and the second fixing part 101B have shapes that are symmetrical in the transverse direction in Fig. 14. Thus, only the first fixing part 101A will be described herein. Fig. 15 is a view for describing the structure of the first fixing part 101A and is a cross-sectional view taken along the line B-B illustrated in Fig. 14. As illustrated in Figs. 14 and 15, the first fixing part 101A includes a frame 101, a support 102, and an air discharge part 103.

The frame 101 has a semicylindrical shape obtained by cutting a cylinder along a plane containing a central axis. The frame 101 includes: a first end 101a, which is one end of a semicylinder and is an end on a side that contacts the skin of a living body; and a second end 101b, which is another end of the semicylinder. The first end 101a has a semicircular shape and has an opening on the inside, and the second end 101b is made of a semicircular flat plate. Thus, the frame 101 has a shape in which a face thereof on the side that contacts the skin of a living body is open.

The frame 101 includes an opening 101c formed on a curved side surface of the semicylinder and in a region where the air discharge part 103 (described later) is installed. Furthermore, the frame 101 includes a groove 101d formed on a flat side surface of the semicylinder so as to be recessed inward.

The support 102, in an inner part of the frame 101, is formed between the first end 101a and the second end 101b in the axial direction (vertical direction in Fig. 15) of the semicylindrical shape of the frame 101. The support 102 is made of a semicircular flat plate having a radius of the same length as an inner diameter of the frame 101. The support 102 has a plurality of air intake holes 102a formed therein that penetrate in the axial direction of the semicylindrical shape of the frame 101.

The air discharge part 103 connects a space in the inner part of the frame 101 and the pump 120A to each other. The air discharge part 103 is provided on the side surface of the frame 101. The air discharge part 103 has a cylindrical hole 103a formed therein. The hole 103a is connected at one end thereof to the opening 101c, which is provided in the frame 101. The hole 103a is connected at another end thereof to the pump 120A, as illustrated in Fig. 14.

As illustrated in Fig. 14, the air discharge part 103 of the first fixing part 101A is connected to the pump 120A, and the air discharge part 103 of the second fixing part 101B is connected to the pump 120B.

The attachment part 110 is located between the first fixing part 101A and the second fixing part 101B. The attachment part 110 includes a frame 111, a guide part 112, a support 113, and an air discharge part 114.

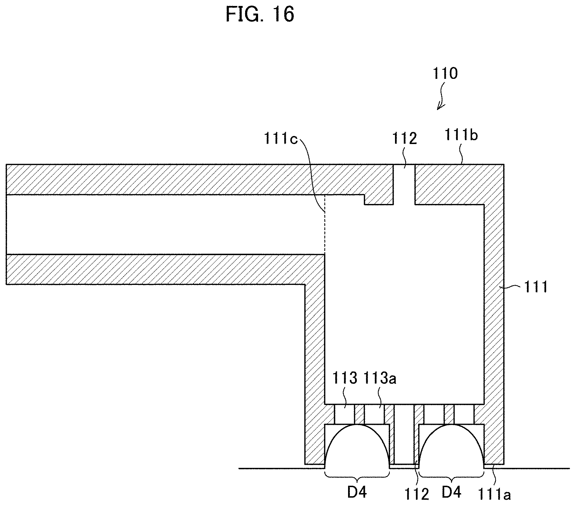

The frame 111 has a tubular shape. The frame 11 includes: a first end 111a, which is one end of a tube and is an end on a side that contacts the skin of a living body; and a second end 111b, which is another end of the tube (see Fig. 16). The first end 111a is shaped to have an opening on the inside, and the second end 111b is made of a flat plate. Thus, the frame 111 has a shape in which a face thereof on the side that contacts the skin of a living body is open.

Further, the frame 111 includes an opening 111c formed on a side surface of a cylinder and in a region where an air discharge part 114 (described later) is installed (see Fig. 16).

The guide part 112 is a member for guiding movement of a needle 23. The guide part 112 has a cylindrical shape. The cylindrical shape is coaxial with the cylindrical shape of the frame 11. The guide part 112 has a through-hole 112a which is formed so as to extend in an axial direction (vertical direction in Fig. 16) of the tube and through which the needle 23 passes.

The support 113, in an inner part of the frame 111, is formed between the first end 111a and the second end 111b in the axial direction (vertical direction in Fig. 16) of the cylindrical shape of the frame 111. The support 113 is made of a flat plate. The support 113 has a plurality of air intake holes 113a formed therein.

The air discharge part 114 is provided on the side surface of the frame 111. The air discharge part 114 has a cylindrical hole 114a formed therein. As illustrated in Fig. 14, the hole 114a has (i) one end that is connected to an opening 111c provided in the frame 111 and (ii) the other end that is connected to the pump 120C.

The following description will discuss an example of a puncture method in which the puncture assisting tool 100 is used. First, the puncture assisting tool 100 is brought into contact with skin S of a living body. Next, the pump 120A and the pump 120B are driven. This reduces pressure inside the first fixing part 101A and pressure inside the second fixing part 101B.

The reduction in pressure inside the frame 11 of the first fixing part 10 1A results in a state in which a region (first region) of the skin S of the living body inside the first end 101a of the frame 101 of the first fixing part 101A is attached to the frame 101 of the first fixing part 101A. Similarly, the reduction in pressure inside the frame 101 of the second fixing part 101B results in a state in which a region (second region) of the skin S of the living body inside the first end 101a of the frame 101 of the second fixing part 101B is attached to the frame 11 of the second fixing part 101B.

Subsequently, in the above states, the first fixing part 101A and the second fixing part 101B are moved in directions in which the first fixing part 101A and the second fixing part 101B are away from each other. This causes a region (third region) of the skin S of the living body between the first region and the second region to be pulled, so that the third region is tensioned.

Then, the pump 120C is driven. This sucks air inside the frame 111 of the attachment part 110. This reduces pressure inside the frame 111 of the attachment part 110. Note that since the support 113 of the frame 111 of the attachment part 110 has the plurality of air intake holes 113a formed therein, it is possible to reduce the pressure in the whole inner part of the frame 111.

Fig. 16 is a cross-sectional view taken along the line C-C of Fig. 14 and illustrating a state in which pressure inside the frame 111 of the attachment part 110 is reduced during puncture in which the puncture assisting tool 100 is used. The reduction in pressure inside the frame 111 of the attachment part 110 results in a state in which a region (region D4 illustrated in Fig. 16) of the skin S of the living body inside the first end 111a of the frame 111 of the attachment part 110 is attached to the inside of the frame 111 as illustrated in Fig. 16.

Next, a syringe (not illustrated) is moved toward the skin S so that the needle 23 is inserted into the skin S of the living body. Here, according to the puncture assisting tool 100 of Embodiment 5, the region into which the needle 23 is inserted in the skin S is in the state of being tensioned by the first fixing part 101A and the second fixing part 101B. Thus, the amount of depression of the skin S by the puncture with the needle 23 is smaller as compared with the amount of depression of the skin S by the puncture with the needle 23 in Embodiment 1. As a result, it is possible to further increase shear stress applied to the skin S by the needle 23. This makes it possible to further increase the chance of inserting the needle 23, which is easily buckled, into the skin S without causing the buckling of the needle 23.

Moreover, according to the puncture assisting tool 100, the needle 23 is guided, during the puncture, by the guide part 112 provided in the attachment part 110. This makes it possible to prevent buckling of the needle 23. In other words, it is possible to use the needle 23 having a small outer diameter. As a result, it is possible to prevent stimulation of pain spots of a living body.

Further, according to the puncture assisting tool 100, the guide part 112 is located inside a region of the attachment part 110 to which region the skin S is attached. This allows a place where the needle 23 is inserted into the skin S of the living body to be a region of the skin S inside the attachment part 110 region.

Aspects of the present invention can also be expressed as follows:
A puncture assisting tool of an aspect of the present invention includes: an attachment part that is attached to skin; and a guide part that is located inside a region of the attachment part in which region the attachment part is attached to the skin, the guide part being provided with a through-hole through which a needle passes and guiding movement of the needle.

The puncture assisting tool of an aspect of the present invention can be configured such that the attachment part suction-attracts the skin.

The puncture assisting tool of an aspect of the present invention can be configured such that the attachment part sticks or adheres to the skin.

The puncture assisting tool of an aspect of the present invention can be configured such that the attachment part has a frame that surrounds the region in which the attachment part is attached to the skin and that has an inner part in which pressure is reduced, and a position of a skin-side end of the frame and a position of a skin-side end of the guide part are aligned with each other.

The puncture assisting tool of an aspect of the present invention can be configured such that the attachment part has a support that supports the skin which has been sucked into the attachment part.

The puncture assisting tool of an aspect of the present invention can be configured to further include a suction device that sucks air inside the attachment part.

A puncture instrument of an aspect of the present invention includes: the above puncture assisting tool; a syringe that has a needle and serves as a suction device; and an air flow part that connects a space inside the attachment part and a space inside the syringe.

The puncture instrument of an aspect of the present invention includes: the above puncture assisting tool; a first syringe that includes a needle; a piston that is stored inside the first syringe; a second syringe that serves as a suction device; a plunger a part of which is stored inside the second syringe; and an air flow part that connects a space inside the attachment part and a space inside the second syringe, wherein the plunger and the piston are connected to each other via a connecting member, and when the plunger is pulled out, air inside the attachment part is sucked by the second syringe, and then the piston is pulled up via the connecting member.

A puncture method of an aspect of the present invention is a puncture method in which any of the above puncture assisting tools is used, the puncture method including: inserting a needle into the skin while the attachment part continues to be in a state of being attached to the skin.

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

- 1, 1A, 1B: puncture instrument
- 10, 30, 100: puncture assisting tool
- 11: frame (attachment part)
- 11a, 101a, 111a: first end (skin-side end)
- 12, 23, 112: guide part
- 12a, 31a, 112a: through-hole
- 12b: third end (skin-side end)
- 13, 113: support
- 23: needle
- 24, 44: second syringe (suction device)
- 28: tube (air flow part)
- 32: adhesive (attachment part)
- 110: attachment part
- 120C: pump (suction device)
- S: skin

## Claims

1. A puncture assisting tool comprising:
an attachment part that is attached to skin; and
a guide part that is located inside a region of the attachment part in which region the attachment part is attached to the skin, the guide part being provided with a through-hole through which a needle passes and guiding movement of the needle.

2. The puncture assisting tool as set forth in claim 1, wherein the attachment part suction-attracts the skin.

3. The puncture assisting tool as set forth in claim 1, wherein the attachment part sticks or adheres to the skin.

4. The puncture assisting tool as set forth in claim 1, wherein the attachment part has a frame that surrounds the region in which the attachment part is attached to the skin and that has an inner part in which pressure is reduced, and
a position of a skin-side end of the frame and a position of a skin-side end of the guide part are aligned with each other.

5. The puncture assisting tool as set forth in claim 2 or 4, wherein the attachment part has a support that supports the skin which has been sucked into the attachment part.

6. The puncture assisting tool as set forth in claim 2, 4, or 5, further comprising a suction device that sucks air inside the attachment part.

7. A puncture instrument comprising:
a puncture assisting tool recited in claim 6;
a syringe that has a needle and serves as a suction device; and
an air flow part that connects a space inside the attachment part and a space inside the syringe.

8. A puncture instrument comprising:
a puncture assisting tool recited in claim 6;
a first syringe that includes a needle;
a piston that is stored inside the first syringe;
a second syringe that serves as a suction device;
a plunger a part of which is stored inside the second syringe; and
an air flow part that connects a space inside the attachment part and a space inside the second syringe,
wherein the plunger and the piston are connected to each other via a connecting member, and
when the plunger is pulled out, air inside the attachment part is sucked by the second syringe, and then the piston is pulled up via the connecting member.

9. A puncture method in which a puncture assisting tool recited in any one of claims 1 to 6 is used, said puncture method comprising:
inserting a needle into the skin while the attachment part continues to be in a state of being attached to the skin.
